# EUROPEAN PATENT SPECIFICATION

(11) **EP 0 322 571 B1**
(45) Date of publication and mention of the grant of the patent: **03.02.1993**
(21) Application number: 88119814.7
(22) Date of filing: 28.11.1988
(51) Int. Cl.: C12P 13/00

(54) **Method for manufacturing 2-amino-2-deoxy-D-mannitol**
Verfahren zur Herstellung von 2-Amino-2-deoxy-D-mannit
Procédé pour la préparation de 2-amino-2 déoxy mannitol

(30) Priority: 28.11.1987 JP 300600/87
(43) Date of publication of application: 05.07.1989
(73) Proprietor: NIPPON SHINYAKU COMPANY, LIMITED, Minami-ku Kyoto-shi Kyoto 601 (JP)
(72) Inventor: Sugiyama, Makoto, Kyoto (JP); Ezure, Yoji, Otsu-shi 520-21 (JP); Ojima, Nobutoshi, Moriyama City Shiga Pref. (JP); Seto, Takashi Room 202, Ukyo-ku Kyoto (JP); Nakamura, Teruya, Kusatsu City (JP); Itoh, Manabu, Otsu City (JP)
(74) Representative: Wey, Hans-Heinrich, Dipl.-Ing.

(56) References cited:
- GB-A- 1 524 493
- GB-A- 2 009 598
- US-A- 4 339 585
- CHEMICAL ABSTRACTS, vol. 103, 1985, page 455, abstract no. 4923w, Columbus, Ohio, US; Y. EZURE et al.: "Moranoline (1-deoxynojirimycin) fermentation and its improvement", & AGRIC. BIOL. CHEM. 1985, 49(4), 1119-25

## Description

The present invention relates to a method for manufacturing 2-amino-2-deoxy-D-mannitol which is the useful substance, as described below.

More particularly, the present invention relates to a method for manufacturing 2-amino-2-deoxy-D-mannitol which comprises culturing a microorganism belonging to the genus Streptomyces and capable of producing 2-amino-2-deoxy-D-mannitol in a medium and isolationg 2-amino-2-deoxy-D-mannitol from the obtained culture broth.

2-Amino-2-deoxy-D-mannitol in accordance with the present invention is not only per se useful as a cosmetic to be formulated (Japanese Published Unexamined Patent Application No. 212421/1984) or as a starting material for preparing X ray contrast media (Japanese Published Unexamined Patent Application No. 128346/1977), but also a useful substance having a specific activity that 2-amino-2-deoxy-D-mannitol enhances an yield of moranoline in the production of moranoline, which exhibits inhiting action of blood sugar increase after having meals and is expected as a remedy for diabetes mellitus, (Japanese Published Examined Patent Application No. 009919/1981) by microorganisms belonging to the genus Streptomyces.

### (Prior Art)

2-Amino-2-deoxy-D-mannitol is a known compound which has been hitherto prepared by reducing N-acetylamino-2-deoxy-D-mannose with sodium borohydride, etc. to convert into N-acetylamino-2-deoxy-D-mannitol and then hydrolyzing N-acetylamino-2-deoxy D-mannitol with an alkali or an acid.

### (Problems to be solved by the Invention)

According to the preparation method described above, however, the method involves defects that N-acetylamino-2-deoxy-D-mannose used as a starting material is expensive, resources are poor, complicated steps for synthesis and isolation are required, and the like.

As a result of investigations on a method for manufacturing 2-amino-2-deoxy-D-mannitol by fermentation over a wide range, the present inventors have found the fact that a microorganism belonging to the genus Streptomyces can produce 2-amino-2-deoxy-D-mannitol in a free form in large quantities and have come to accomplish the present invention.

A representative example of the microorganism in accordance with the present invention is Streptomyces lavendulae SEN-158 (hereafter referred to as "SEN-158 strain") isolated from a soil in Sapporo City, Japan by the present inventors.

SEN-158 strain has been deposited in the Fermentation Research Institute of the Agency of Industrial Science & Technology of Japan under Accession Number of FERM P-4301. Its bacteriologial properties are described in Japanese Published Unexamined Patent Application No. 084094/1979. Further this strain has been deposited in the American Type Culture Collection, Rockville Md., under Accession Number of ATCC 31434.

In the present invention, any other strain is usable as far as it belongs to the genus Streptomyces and is capable of producing 2-amino-2-deoxy-D-mannitol. Further, artificial mutants obtained by applying to these strains variation means conventionally used such as a treatment for irradiating with ultraviolet rays or 60Co, etc., a variation treatment with a variation inducer such as nitrogen mustard, azaserine, nitric acid, nitrosoguanidine or 2-aminopurine, etc., transduction, transformation, cell fusion, etc. and naturally occurring variants are also suited for the purpose of the present invention.

The present invention can be practiced by using the 2-amino-2-deoxy-D-mannitol-producing strain and culturing the strain in a conventional manner for culturing Actinomycetes.

A medium can be liquid or solid but shake culture or submerged culture in liquid medium can ordinarily be used.

The medium can be any one that is suited for growth of Actinomycetes and capable of producing 2-amino-2-deoxy-D-mannitol.

As the carbon sources, there can be used glucose, galactose, mannitol, sucrose, maltose, glycerine, dextrin, starch, or the like.

As the nitrogen sources, there can be used soybean powders, peptone, yeast extract, meat extract, corn steep liquor, ammonium chloride, ammonium sulfate, ammonium nitrate, urea, etc.

In addition, supplementation of sodium chloride, potassium chloride, calcium carbonate, various phosphates or the like in a suitable amount can provide good results. Furthermore, a suitable amount of iron, magnesium, etc. may also be added.

If necessary, organic or inorganic compounds, vitamins, etc. can be supplemented to accelerate growth of the microorganisms used or production of 2-amino-2-deoxy-D-mannitol.

In case that foaming is remarkable during fermentation, a defoaming agent may be appropriately added.

Conditions for incubation such as a pH value of the medium, incubation temperature and the like can be appropriately varied within such a range that produces 2-amino-2-deoxy-D-mannitol. For example, in shake culture or submerged culture, it is desired to culture at pH of from 6 to 9 at an incubation temperature of from approximately 20 to 35°C, preferably 25 to 30°C.

A time period for incubation varies depending upon scale of culture and other conditions but it is generally sufficient for 2 to 20 days.

After incubation, the cells are separated and the product is isolated from the obtained culture broth and purified.

In order to isolate and purify the substance of the present invention from the culture broth , conventional technique used for isolating and purifying microorganism metabolites from the culture broth can be used.

For example, adsorption and desorption with various adsorbents (e.g., silica gel, alumina, activated charcoal, ion exchange resin, etc.), chromatography, partition chromatography, fractional crystallization, recrystallization, etc., can be used singly or in combination.

Hereafter the present invention is described in more detail by referring to an example and a test example below.

### Example 1

In a 500 ml Erlenmeyer flask, 100 ml medium containing (8% of soluble starch, 1% of soybean powders, 1% of yeast extract, 0.05% of potassium chloride, 0.05% of magnesium sulfate, 0.5% of sodium chloride and 0.2% of sodium nitrate, pH 7.2) was charged and sterilized. Several platinum loops of SEN-158 strain were inoculated to the medium from the slant followed by shake culture at 27°C for 3 days to give a preculture solution. This preculture solution, 300 ml, was inoculated on 15 liters of culture solution (components are the same as in the preculture solution) charged in a 30 liter volume jar fermenter followed by culture at 27°C for 11 days.

As a defoaming agent, NISSAN DISFOAM CB-442 was used; an aeration rate and an agitation speed were 20 liters/min. and 300 rpm, respectively.

After 12.9 liters of the obtained culture broth was centrifuged at 9000 rpm for 20 minutes, the obtained supernatant was passed through a column packed with strongly acidic ion exchange resin Dowex 50W x 2 (H⁺) (1 liter). After thoroughly washing with water, elution was performed with 1 N ammonia water.

The eluate was concentrated under reduced pressure. The concentrate was passed through strongly basic ion exchange resin Diaion SA-11A (OH⁻) (500 ml) followed by eluting with water. The eluate was combined with the washing liquid. The mixture was concentrated under reduced pressure. The concentrate was allowed to stand at 5°C for several days. The formed crystals were collected and recrystallized from 20% hydrated ethanol to give 1 g of 2-amino-2-deoxy-D-mannitol. Physical Properties of this product are as follows.
Melting point: 161-163°C

| Elemental analysis (C₆H₁₅NO₅) | | | |
|---|---|---|---|
| Calcd. (%) | C: 39.77 | H: 8.34 | N: 7.73 |
| Found (%) | C: 39.62 | H: 8.17 | N: 7.81 |

Specific rotary power [α] 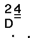 +4.0° (C= 1%, water)
¹³C-NMR ppm; (D₂O, internal standard; methanol 49.8 ppm)
53.84 64.05 64.31 70.73 71.29 71.85
¹H-NMR ppm; (D₂O, internal standard; DSS)
2.98-3.12 (1H, m), 3.56-3.90 (7H, m)
The structure of this product was confirmed by the fact that the properties were fully identical with those of a compound synthesized by reducing N-acetylamino-2-deoxy-D-mannitol with sodium borohydride and then hydrolyzing it with hydrochloric acid.

### Test Example

### Effect of 2-amino-2-deoxy-D-mannitol on moranoline production

Variant GC-148 of newly isolated moranoline-producing strain MB-733 belonging to the genus Streptomyces was inoculated on a medium shown below. After shake culture at 27°C for 7 days, 10 ml of the culture broth was centrifuged. The supernatant was passed through a column packed with strongly acidic ion exchange resin Dowex 50W x 2 (H⁺) (1 liter). After thoroughly washing with water, elution was performed with 0.5 N ammonia water. After the eluate was concentrated to dryness under reduced pressure, the residue was dissolved in 1 ml of water. The solution was subjected to high performance liquid chromatography to quantitatively determine moranoline.

Conditions for high performance liquid chromatography were as follows: column, Nucleosil 5NH₂; developing solvent, acetonitrile-water = 7 : 3; detection, differential refractometer.

Medium A: 2% of soluble starch, 1% of soybean powders, 1% of yeast extract, 0.05% of potassium chloride, 0.05% of hydrated magnesium sulfate, 0.5% of sodium chloride, 0.2% of sodium nitrate and 0.35% of calcium carbonate; pH 7.0
Medium B: medium obtained by supplementing 1% of 2-amino-2-deoxy-D-mannitol to Medium A
The results are shown in the table below.

| | pH | Moranoline (µg/ml) |
|---|---|---|
| Medium A | 8.6 | 981 |
| Medium B | 7.6 | 4428 |

The moranoline-producing effect of 2-amino-2-deoxy-D-mannitol in accordance with the present invention is clear from the results.

## Claims

1. A method for manufacturing 2-amino-2-deoxy-D-mannitol which comprises culturing a microorganism belonging to the genus Streptomyces and capable of producing 2-amino-2-deoxy-D-mannitol in a medium and isolating 2-amino-2-deoxy-D-mannitol from the obtained culture broth.

## Patentansprüche

1. Verfahren zum Herstellen von 2-Amino-2-desoxy-D-mannitol durch Kultivierung eines zur Gattung Streptomyces gehörenden und zur Erzeugung von 2-Amino-2-desoxy-D-mannitol in einem Medium fähigen Mikroorganismus und Isolieren von 2-Amino-2-desoxy-D-mannitol aus dem erhaltenen Kulturmilieu.

## Revendications

1. Procédé pour la préparation de 2-amino-2-désoxy-D-mannitol, ledit procédé comprenant la culture dans un milieu d'un microorganisme appartenant au genre Streptomyces et l'isolation du 2-amino-2-désoxy-D-mannitol du milieu de culture ainsi obtenu.
